# EUROPEAN PATENT APPLICATION

(11) **EP 4 847 549 A1**
(43) Date of publication of application: **02.12.2026**
(21) Application number: 25914947.4
(22) Date of filing: 14.11.2025
(51) Int. Cl.: C08G 65/333, C09J 123/08

(54) **COUPLED MACROMOLECULAR LIGHT CONVERSION AGENT AND PREPARATION METHOD THEREFOR, ENCAPSULATION COMPOSITION, ENCAPSULATION ADHESIVE FILM, AND PHOTOVOLTAIC MODULE**

(30) Priority: 30.12.2024 CN 202411974343
(71) Applicant: Hangzhou First Applied Material Co., Ltd., Hangzhou, Zhejiang 311300 (CN)
(72) Inventor: XU, Li, Hangzhou, Zhejiang 311300 (CN); TANG, Guodong, Hangzhou, Zhejiang 311300 (CN); WEI, Mengjuan, Hangzhou, Zhejiang 311300 (CN); HOU, Hongbing, Hangzhou, Zhejiang 311300 (CN); ZHOU, Guangda, Hangzhou, Zhejiang 311300 (CN)
(74) Representative: Novagraaf Technologies
(86) International application number: PCT/CN2025/135186
(87) International publication number: WO 2026/144595

(57) **Abstract**

Provided is a coupled macromolecular light-conversion agent and a preparation method therefor, an encapsulation composition, an encapsulation adhesive film and a photovoltaic module. Provided in the present invention is a coupled macromolecular light-conversion agent comprising a first light-conversion agent and a second light-conversion agent, wherein the first light-conversion agent and the second light-conversion agent are coupled and connected, and the first light-conversion agent and the second light-conversion agent are each independently a benzotriazole compound having a structure as shown in formula (I). The coupled macromolecular light-conversion agent provided in the present invention is a coupled product of a first light-conversion agent and a second light-conversion agent of a benzotriazole compound. The number-average molecular weight of the coupled macromolecular light-conversion agent is significantly improved, and not only can the compatibility with a matrix be significantly improved, but also the number-average molecular weight is significantly increased, so that the coupled macromolecular light-conversion agent can effectively inhibit its transfer in a adhesive film, and the light conversion performance is significantly improved.

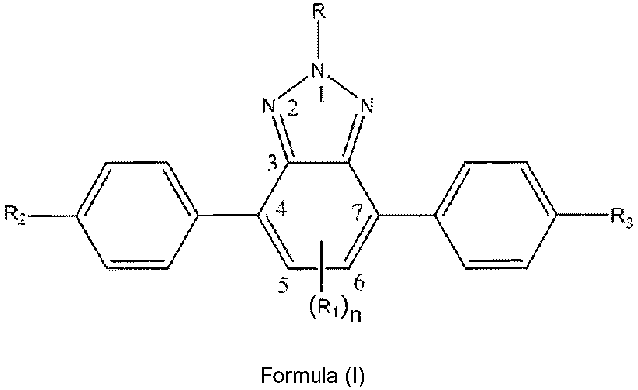

## Description

### Cross-Reference to Related Application

This invention claims priority to Chinese Patent Application No. 202411974343.8, filed with the Chinese Patent Office on December 30, 2024 and entitled "Coupled macromolecular light-conversion agent and preparation method therefor, encapsulation composition, encapsulation adhesive film and photovoltaic module", which is incorporated herein by reference in its entirety.

### Technical Field

The present invention relates to the technical field of a light conversion agent, and specifically relates to a coupled macromolecular light-conversion agent and a preparation method therefor, a packaging composition, a packaging adhesive film and a photovoltaic module.

### Background

An adhesive film containing a light conversion agent can convert ultraviolet light with a low utilization rate of a solar cell into visible light with a high utilization rate, thereby expanding the spectral response range of the solar cell, improving the photoelectric conversion efficiency of the solar cell, and at the same time protecting a passivation layer of the solar cell from being damaged by ultraviolet light, thereby prolonging the service life of the solar cell. According to the structure of a solar cell in practical applications, a light conversion material can generally be selected to be mixed with an ethylene-vinyl acetate copolymer (EVA) resin, an ethylene-a olefin copolymer (POE) resin, a polyvinyl butyral (PVB) resin, liquid silicone rubber, etc., so as to construct an encapsulation adhesive film or encapsulation glue having a light conversion function, and apply same to a light receiving surface of a solar cell assembly. At present, a commercially available light conversion EVA adhesive film product is sold, but the effect thereof is still poor, and the main problem lies in that an organic small molecule light conversion agent will migrate, and migrates slowly from a light receiving surface encapsulation adhesive film to an encapsulation adhesive film of a backlight surface, thereby causing a decrease in light conversion performance; however, the inorganic light conversion agent is generally incompatible with the matrix resin and has a phase difference, causing the light transmittance of the encapsulation adhesive film to decrease.

The prior art discloses that a conversion agent is introduced into a polymer matrix by means of grafting or copolymerization so as to prevent the light conversion agent from being transferred. However, whether grafting or copolymerization, a chemical structure change needs to be made on a resin layer, which has relatively high requirements on devices, processes, etc. Furthermore, with regard to grafting, a grafting rate of 100% cannot be achieved, resulting in a by-product remaining in the system. For copolymerization, there is a problem of reactivity ratio, and huge adjustments are required for the catalyst, reaction medium and polymerization process.

In view of this, the present invention is specially proposed.

### Summary

The main purpose of the present invention is to provide a coupled macromolecular light-conversion agent and a preparation method therefor, an encapsulation composition, an encapsulation adhesive film and a photovoltaic module, to solve the problem in the prior art that the transfer of the light-conversion agent is avoided by introducing the light-conversion agent into a polymer matrix by means of grafting, as a result, a by-product remains in the system, and the light-conversion agent is prevented from being transferred by introducing the light-conversion agent into the polymer matrix by copolymerization. In this case, there is a problem of reactivity ratio, and the catalyst, reaction medium, and polymerization need to be greatly adjusted.

In order to achieve the described object, according to one aspect of the present invention, provided is a coupled macromolecular light-conversion agent. The coupled macromolecular light-conversion agent includes a first light-conversion agent and a second light-conversion agent, and the first light-conversion agent and the second light-conversion agent are coupled, wherein the first light-conversion agent and the second light-conversion agent are each independently a benzotriazole compound, and the benzotriazole compound has a structure as shown in formula (I): 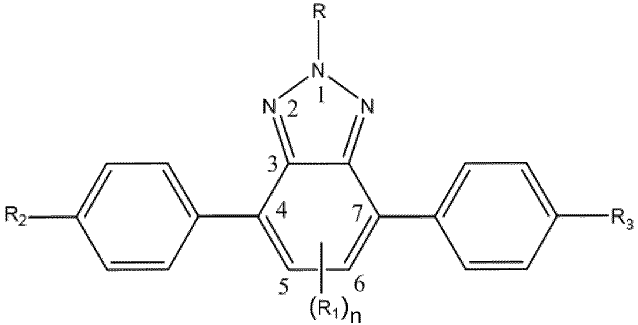 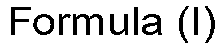 wherein n is 1 or 2, R₁ is selected from H, a substituted or unsubstituted C1 to C20 alkyl group or a first group; R₂, R₃ are each independently selected from H, a substituted or unsubstituted C1 to C20 alkyl group; the general formula of R and the first group is R₅-R₄*, in which R₅ represents a terminal group, and R₅ is selected from at least one of a mercapto group, a carboxyl group, a hydroxyl group, a primary amino group, a secondary amino group, an epoxy group, a vinyl group and an acetylene group; R₄ is selected from a direct bond, a substituted or unsubstituted C1-C20 alkyl group, and C and H in the alkyl group may be optionally substituted with O.

Further, R is selected from *CH₂-(CH₂)₅-CH=CH₂, *CH₂-CH₂COO-CH=CH₂, *CH₂-(CH₂)₂-COOH, *CH₂-(CH₂)₂-CH₂OH, *CH₂-(CH₂)₃-SH, *CH₂-(CH₂)₃-NH₂.

Further R₁ is selected from H, C1-C6 alkyl, *CH₂-(CH₂)₅-CH=CH, CH₂-(CH₂)₂-COOH, *CH₂-(CH₂)₂-CH₂OH, *CH₂-(CH₂)₃-NH₂, *CH₂-(CH₂)₃-SH.

Further, R₂ and R₃ are each independently selected from H or C1-C6 alkyl.

Further, the number-average molecular weight of the coupled macromolecular light-conversion agent is from 1000 to 100000 g/mol.

Further, the maximum absorption wavelength of the coupled macromolecular light-conversion agent is between 300-400 nm, and the maximum emission wavelength is greater than 400 nm.

Further, the first light conversion agent and the second light conversion agent are each independently selected from at least one of the following compounds:

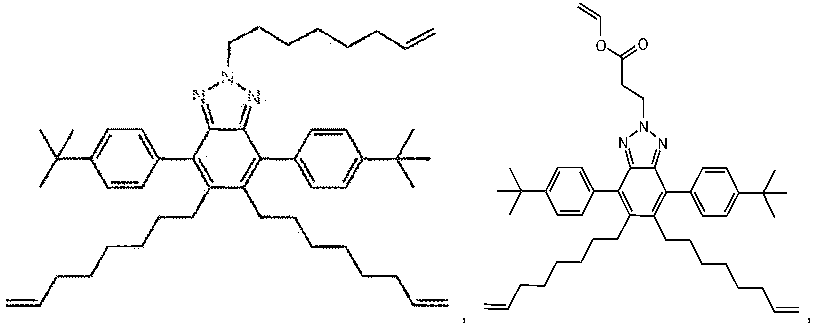

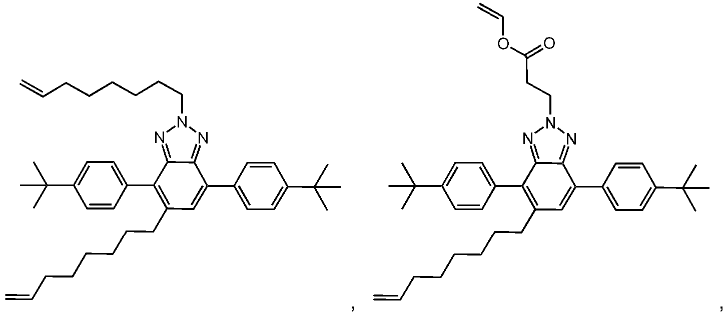

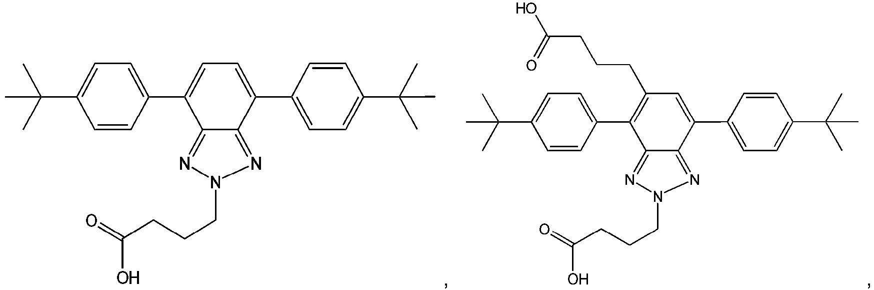

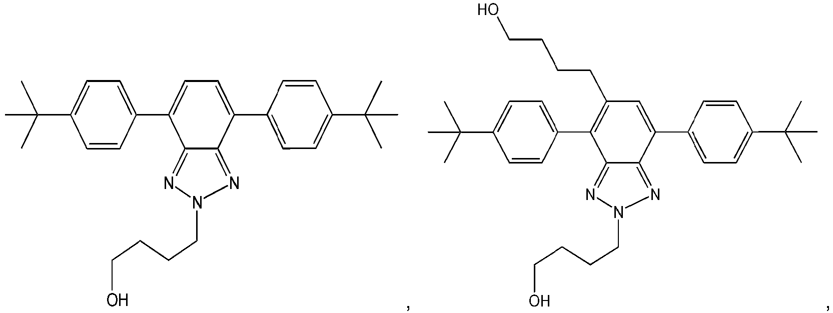

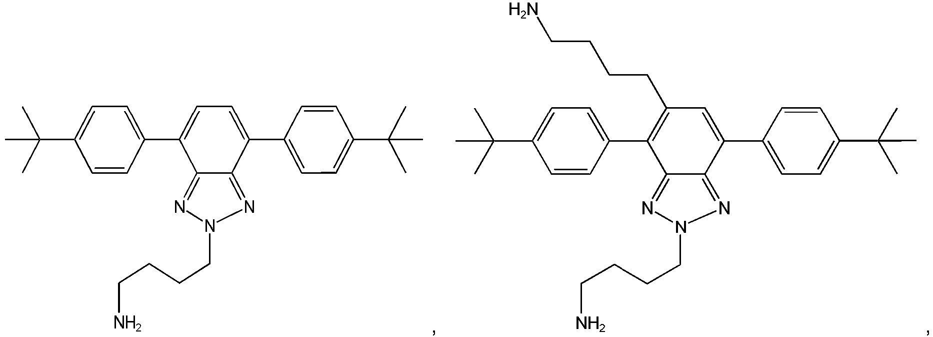

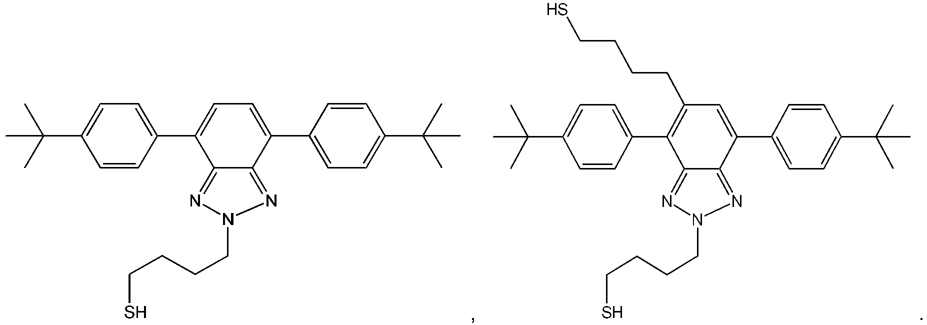

According to a second aspect of the present invention, there is further provided a method for preparing the coupled macromolecular light-conversion agent provided in the first aspect. The preparation method comprises: step S1, dissolving a first light-conversion agent and a second light-conversion agent in a solvent respectively, so as to obtain a first light-conversion agent solution and a second light-conversion agent solution; step S2: mixing the first light-conversion agent solvent solution, the second light-conversion agent solution, a coupling agent and an optional initiator for a coupling reaction to obtain a coupled macromolecular light-conversion agent.

Further, the coupling agent is selected from at least one of bis(2-mercaptoethyl) ether, 4,4'-bis(mercaptomethyl) biphenyl, C2-C10 diols, polyethylene glycol, polytetrahydrofuran, poloxamer, polyvinyl alcohol, terminal (poly) hydroxy polybutadiene, terminal (poly) hydroxy polyethylene, terminal hydroxy liquid nitrile rubber, C2-C10 dicarboxylic acid, terminal carboxy polybutadiene, terminal carboxy polyethylene, terminal carboxy nitrile rubber, polyacrylic acid, polymethacrylic acid, C2-C10 diamines, polyether amine, terminal amino liquid nitrile rubber, terminal amino polybutadiene, terminal amino polyethylene, 1,4-butanediol glycidyl ether, glycidyl ether, epoxy resin, C4-C18 diene, polybutadiene, ethylene-propylene-diene terpolymer, and styrene-butadiene rubber.

According to a third aspect of the present invention, also provided is an encapsulation composition. The encapsulation composition comprises the following components in parts by mass: 100 parts of a matrix resin, 0.01-1 parts of a macromolecular light conversion agent, 0-1 parts of a hindered amine light stabilizer, 0-2 parts of an organic peroxide, 0-3 parts of a crosslinking coagent, and 0-3 parts of a tackifier; the macromolecular conversion agent is a coupled macromolecular light-conversion agent provided in the first aspect.

Further, the matrix resin comprises at least one of an EVA resin, a POE resin, a PVB resin, an ethylene-acrylate resin, an ethylene-methacrylate resin and liquid silicone rubber.

Further, hindered amine light stabilizer includes hexadecyl 3,5-di-tert-butyl-4-hydroxy-benzoate, tris(1,2,2,6,6-pentamethyl-4-piperidinyl) phosphite, bis(2,2,6,6-tetramethylpiperidinol) sebacate, bis-1-decanoxy-2,2,6,6-tetramethyl-piperidin-4-ol sebacic acid ester, polymers of succinic acid and 4-hydroxy-2,2,6,6-tetramethyl-1-piperidinol, N, at least one of polymers of N'-bis(2,2,6,6-tetramethyl-4-piperidyl)1,6-hexanediamine, morpholine-2,4,6-trichloro-1,3,5-triazine.

Further, the organic peroxide includes at least one of diacyl peroxide, dialkyl peroxide, peroxyester, and peroxyketal.

Further, the crosslinking coagent includes at least one of triallyl isocyanurate, triallyl cyanurate, trimethylolpropane triacrylate, trimethylolpropane trimethacrylate, pentaerythritol triacrylate.

Further, the adhesion promoter includes at least one of γ-aminopropyltriethoxysilane, γ-methacryloxypropyltrimethoxysilane, γ-(2,3-epoxypropoxy)propyltrimethoxysilane, vinyltrimethoxysilane, vinyltriethoxysilane, N-(β-aminoethyl)-γ-aminopropyltrimethoxysilane, γ-glycidoxypropyltrimethoxysilane, 3-aminopropyltrimethoxysilane, vinyltriethoxysilane, vinyltris(β-methoxyethoxy silane), and allyltrimethoxysilane.

According to a fourth aspect of the present invention, there is provided an encapsulation adhesive film, the material of which comprises the coupled macromolecular light-conversion agent provided in the first aspect or the encapsulation composition provided in the third aspect.

According to a fifth aspect of the present invention, there is provided a photovoltaic module, the photovoltaic module including an encapsulation adhesive film, the material of the encapsulation adhesive film including the coupled macromolecular light-conversion agent provided in the first aspect or the encapsulation composition provided in the third aspect.

By applying the technical solution of the present invention, the coupled macromolecular light-conversion agent provided in the present invention is a coupled product of a first light-conversion agent and a second light-conversion agent of a benzotriazole compound. The number-average molecular weight of the coupled macromolecular light-conversion agent is significantly increased, the compatibility with a matrix is significantly improved, the number-average molecular weight is significantly increased, the transfer of the coupled macromolecular light-conversion agent in an encapsulation adhesive film can be effectively inhibited, and the light-conversion performance is significantly improved.

### Detailed Description of the Embodiments

It is important to note that the embodiments of the present disclosure and the characteristics in the embodiments can be combined under the condition of no conflicts. The present disclosure will be described below in detail with reference to embodiments.

As described in the background art of the present invention, the small organic molecule light conversion agent in the commercially available light-converting EVA adhesive film will migrate, and slowly migrates from the packaging adhesive film on the light-receiving surface to the packaging adhesive film on the backlight surface, resulting in a decrease in light-converting performance. The prior art discloses that a conversion agent is introduced into a polymer matrix by means of grafting or copolymerization so as to prevent the conversion of the conversion agent; however, the grafting method cannot achieve a grafting rate of 100%, resulting in that a by-product remains in the system, and the copolymerization method has a problem of competitive polymerization rate; and a catalyst, a reaction medium and a polymerization process all need to be adjusted greatly, which is very difficult. In order to solve these problems, the present invention provides a coupled macromolecular light-conversion agent and a preparation method therefor, an encapsulation adhesive film, an encapsulation composition and a photovoltaic module.

In a typical embodiment of the present invention, provided is a coupled macromolecular light-conversion agent. The coupled macromolecular light-conversion agent comprises a first light-conversion agent and a second light-conversion agent, and the first light-conversion agent and the second light-conversion agent are coupled and connected; the first light-conversion agent and the second light-conversion agent are each independently a benzotriazole compound. The benzotriazole compound has a structure as shown in formula (I):

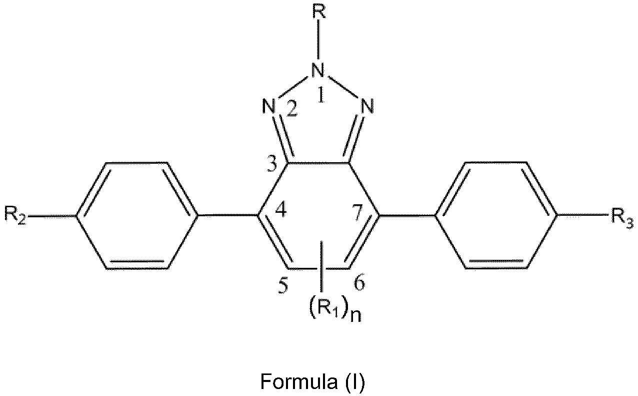

Wherein n is 1 or 2, R₁ is selected from H, a substituted or unsubstituted C1 to C20 alkyl group, or a first group; R₂, R₃ are each independently selected from H, a substituted or unsubstituted C1 to C20 alkyl group; the general formula of R and the first group is each independently R₅-R₄*, in which R₅ represents a terminal group, and R₅ is selected from at least one of mercapto group, carboxyl group, hydroxyl group, primary amino group, secondary amino group, epoxy group, vinyl group and acetylene group; R₄ is selected from a direct bond, a substituted or unsubstituted C1 to C20 alkyl group.

In the present invention, "*" represents a linking bond, alkyl comprises linear alkyl and branched alkyl, which may be linear or branched. Examples of C1-C20 alkyl groups include methyl, ethyl, n-propyl, isopropyl, n-butyl, isobutyl, sec-butyl, tert-butyl, 2-methylbutyl, n-pentyl, sec-pentyl, neopentyl, n-hexyl, neohexyl, n-heptyl, n-octyl, 2-ethylhexyl, trifluoromethyl, pentafluoroethyl, 2,2,2-trifluoroethyl and the like.

In this application, the term "substituted or unsubstituted" refers to substitution with one or more substituents selected from deuterium; a halogen group; nitrile groups; nitro; hydroxy; carbonyl; ester groups; imide groups; amino; phosphinyl oxide; alkoxy; aryloxy; alkylthio; arylthio; alkylsulfonyl; silyl; boron groups; alkyl; cycloalkyl; alkenyl; aryl; aralkyl; aralkenyl; alkylaryl; alkyl amine; aralkylamino; heteroarylamino; arylamino; arylphosphino; or a heterocyclyl containing at least one of N, O and S, either without a substituent, or substituted by a substituent to which two or more of the exemplified substituents are attached, or without a substituent. For example, the term "substituent to which two or more substituents are attached" may be biphenyl. That is, the biphenyl group may be an aryl group, or may be interpreted as a substituent group where two phenyl groups are connected.

The coupled macromolecular light-conversion agent provided in the present invention is a coupled product of a first light-conversion agent and a second light-conversion agent of a benzotriazole compound. The number-average molecular weight of the coupled macromolecular light-conversion agent is significantly improved, and not only can the compatibility with a matrix be significantly improved, but also the number-average molecular weight is significantly increased, so that the coupled macromolecular light-conversion agent can effectively inhibit its transfer in an encapsulation adhesive film, and the light conversion performance is significantly improved.

In some embodiments, R is *CH₂-(CH₂)₅-CH=CH₂, *CH₂-CH₂COO-CH=CH₂, *CH₂-(CH₂)₂-COOH, *CH₂-(CH₂)₂-CH₂OH, *CH₂-(CH₂)₃-SH, *CH₂-(CH₂)₃-NH₂ is more advantageous to simplify the process of preparing coupled macromolecular light-conversion agents.

In some embodiments, R₁ is H, C1-C6 alkyl, CH₂-(CH₂)₅-CH=CH₂, *CH₂-CH₂COO-CH=CH₂, *CH₂-(CH₂)₂-COOH, *CH₂-(CH₂)₂-CH₂OH, *CH₂-(CH₂)₃-SH, *CH₂-(CH₂)₃-NH₂, wherein C1-C6 alkyl is such as methyl, ethyl, n-propyl, isopropyl, and the like.

In some embodiments, R₂ and R₃ are each independently C1-C6 alkyl, such as methyl, ethyl, n-propyl, isopropyl, and the like.

In order to further improve the stability of the coupled macromolecular light-conversion agent **in** the encapsulation adhesive film, the number-average molecular weight of the coupled macromolecular light-conversion agent is preferably 1000-100000 g/mol, such as 1000 g/mol, 2000 g/mol, 5000 g/mol, 8000 g/mol, 10000 g/mol, 20000 g/mol, 30000 g/mol, 50000 g/mol, 60000 g/mol, 80000 g/mol, 90000 g/mol, 100000 g/mol, or a range value consisting of any two numeric values.

In order to further improve the light conversion performance, preferably, the maximum absorption wavelength of the coupled macromolecular light-conversion agent is between 300-400 nm, and the maximum emission wavelength is greater than 400 nm.

In some embodiments, the first light conversion agent and the second light conversion agent are each independently selected from at least one of the following compounds:

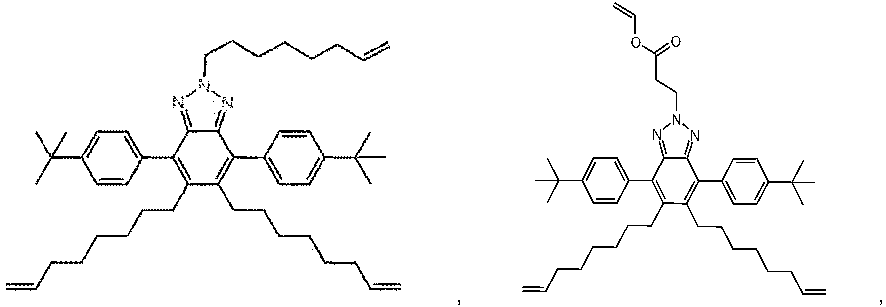

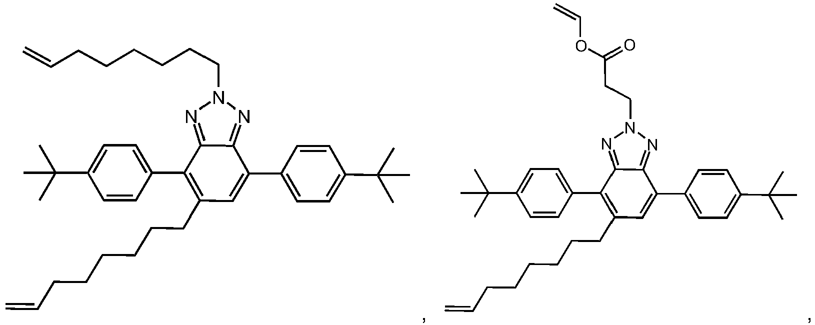

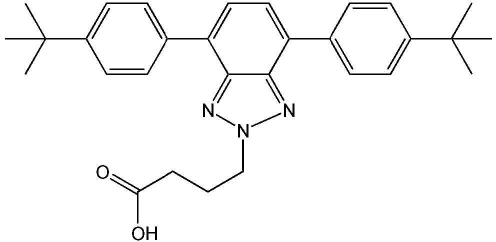

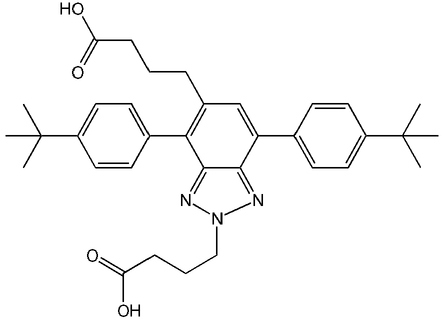

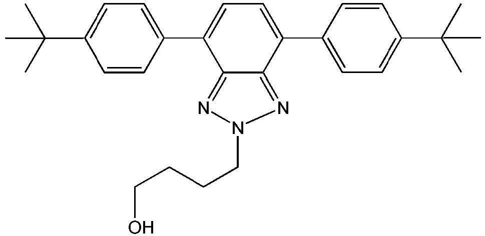

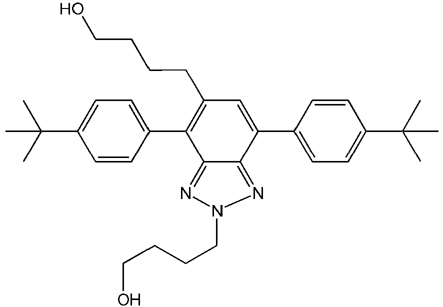

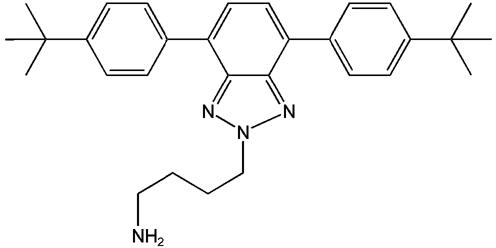

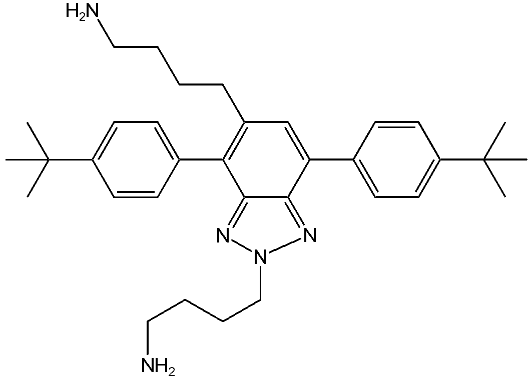

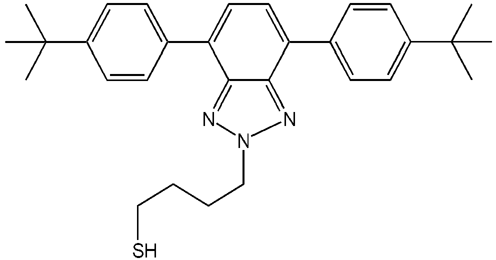

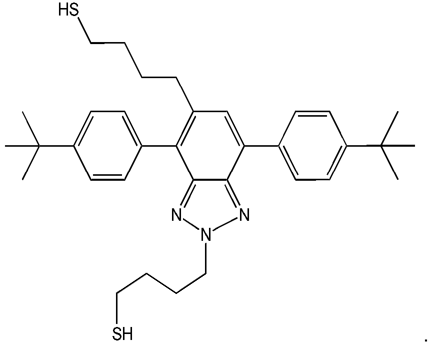

In a second exemplary embodiment of the present invention, there is further provided a method for preparing the above coupled macromolecular light-conversion agent. The preparation method comprises: step S1, respectively dissolving a first light-conversion agent and a second light-conversion agent in a solvent to obtain a first light-conversion agent solution and a second light-conversion agent solution; Step S2: mixing the first light-conversion agent solution, the second light-conversion agent solution, a coupling agent and an optional initiator for a coupling reaction to obtain a coupled macromolecular light-conversion agent.

The preparation method for a coupled macromolecular light-conversion agent provided in the present invention is simple and easy, has a low cost, does not introduce impurities such as by-products, and can effectively guarantee the quality of an encapsulation adhesive film.

In the present invention, the specific type of the coupling agent is not limited, and common coupling agents in the art may include, but are not limited to, any mixture of one or more of bis(2-mercaptoethyl) ether, 4,4'-bis(mercaptomethyl)biphenyl, C2-C10 glycol, polyethylene glycol, polytetrahydrofuran, poloxamer, polyvinyl alcohol, terminal (poly)hydroxypolybutadiene, terminal (poly)hydroxypolyethylene, terminal hydroxy liquid nitrile rubber, C2-C10 dicarboxylic acid, terminal carboxy polybutadiene, terminal carboxy polyethylene, terminal carboxy nitrile rubber, polyacrylic acid, polymethacrylic acid, C2-C10 diamine, polyether amine, terminal amino liquid nitrile rubber, terminal amino polybutadiene, terminal amino polyethylene, 1,4-butanediol glycidyl ether, glycidyl ether, epoxy resin, C4-C18 diene, polybutadiene, ethylene-propylene-diene ternary rubber, and styrene-butadiene rubber.

The above-mentioned "C2-C10 diols" refer to diols containing two hydroxyl groups (-OH), the carbon chain length of which is between 2 and 10 carbon atoms. "C2" represents containing two carbon atoms, while "C10" represents containing ten carbon atoms.

The aforementioned "C2-C10 dicarboxylic acid" refers to an organic acid containing two carboxyl groups (-COOH) with a carbon chain length between 2 and 10 carbon atoms. "C2" represents a moiety containing two carbon atoms, while "C10" represents a moiety containing ten carbon atoms

The above-mentioned "C2-C10 diamines" refer to compounds containing two primary amine groups (-NH₂), the two amino groups being connected to carbon atoms, and the chain length of these carbon atoms ranging from 2 to 10 carbon atoms.

The foregoing "terminal (poly)hydroxypolybutadiene" refers to a polymer containing a hydroxyl (-OH) functional group at a terminal or a plurality of positions of a polybutadiene molecular chain.

The above-mentioned "terminal (poly)hydroxypolyethylene" refers to a polymer containing a hydroxyl (-OH) functional group at a terminal or a plurality of positions of a molecular chain of the polyethylene.

In order to further improve the efficiency of the coupling reaction, preferably, the temperature of the coupling reaction is 0-95°C, and the duration of the coupling reaction is 10-24 h.

Typical without limitation, temperatures for the coupling reaction are, for example, 0°C, 5°C, 10°C, 20°C, 30°C, 40°C, 50°C, 55°C, 60°C, 65°C, 70°C, 75°C, 80°C, 85°C, 90°C, 95°C, or a range value consisting of any two of the foregoing; the time of the coupling reaction is, for example, 10 h, 11 h, 12 h, 13 h, 14 h, 15 h, 18 h, 20 h, 22 h, 24 h or a range of values consisting of any two numbers.

In some specific embodiments, in step S2, the first conversion solution, the second conversion solution, the coupling agent and optional initiator are mixed and reacted at 0-95°C for 10-24 hours to obtain a coupled conversion macromolecule reaction solution, and the coupled conversion macromolecule reaction solution is purified to obtain the coupled conversion macromolecule conversion agent.

Exemplarily, the purification of the coupled conversion macromolecule reaction solution comprises the following steps: adding ethyl acetate to the coupled conversion macromolecule reaction solution for precipitation, then performing solid-liquid separation to obtain a solid reactant, washing the solid reactant multiple times with ethyl acetate, and then performing vacuum drying overnight at 30°C to obtain the coupled conversion macromolecule conversion reagent.

An exemplary procedure for preparing a coupled macromolecule light conversion agent is as follows:

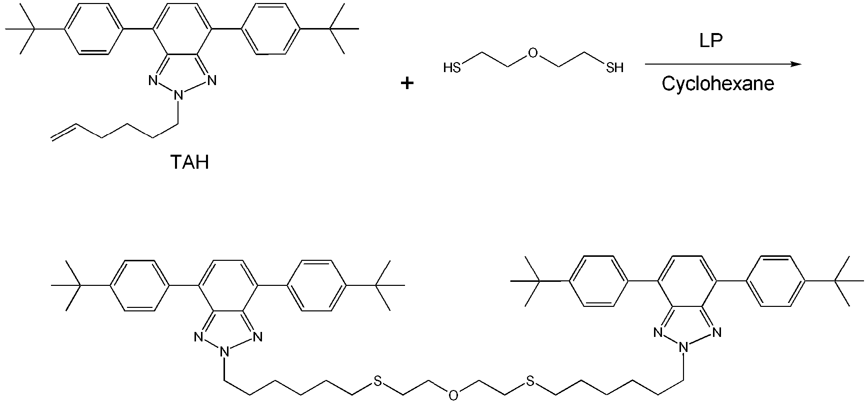

Note: "cyclohexane" means cyclohexane; "LP" refers to lauroyl peroxide.

In a third exemplary embodiment of the present invention, provided is an encapsulation composition, comprising the following components in parts by mass: 100 parts of a matrix resin, 0.01-1 part of a macromolecular light conversion agent, 0-1 part of a hindered amine light stabilizer, 0-2 parts of an organic peroxide, 0-3 parts of a crosslinking coagent, and 0-3 parts of a tackifier; the macromolecular light-conversion agents are coupled macromolecular light-conversion agents provided in the above first exemplary embodiment.

Typically, but not limitatively, in the encapsulation composition provided by the present invention, based on the weight of the matrix resin being 100 parts, the amount of the coupled macromolecular light-conversion agent used is, for example, 0.01 part, 0.02 part, 0.05 part, 0.08 part, 0.1 part, 0.15 part, 0.2 part, 0.3 part, 0.4 part, 0.5 part, 0.6 part, 0.7 part, 0.8 part, 1 part, or a range value consisting of any two numeric values; the amount of the hindered amine light stabilizer used is, for example, 0 part, 0.01 part, 0.02 part, 0.05 part, 0.08 part, 0.1 part, 0.15 part, 0.2 part, 0.3 part, 0.4 part, 0.5 part, 0.6 part, 0.7 part, 0.8 part, 0.9 part, 1 part or a range value consisting of any two of the values; the amount of the organic peroxide used is, for example, 0 part, 0.01 part, 0.02 part, 0.05 part, 0.08 part, 0.1 part, 0.15 part, 0.2 part, 0.3 part, 0.4 part, 0.5 part, 0.6 part, 0.7 part, 0.8 part, 0.9 part, 1 part, 1.1 parts, 1.2 parts, 1.3 parts, 1.4 parts, 1.5 parts, 1.6 parts, 1.7 parts, 1.8 parts, 1.9 parts, 2 parts or a range value consisting of any two of the foregoing values; the amount of the crosslinking coagent used is, for example, 0 part, 0.01 part, 0.02 part, 0.05 part, 0.08 part, 0.1 part, 0.2 part, 0.5 part, 0.8 part, 1.0 part, 1.2 parts, 1.5 parts, 1.8 parts, 2.0 parts, 2.2 parts, 2.5 parts, 2.8 parts, 3.0 parts or a range value consisting of any two of the foregoing values; tackifiers are for example 0 part, 0.01 part, 0.02 part, 0.05 part, 0.08 part, 0.1 part, 0.2 part, 0.5 part, 0.8 part, 1.0 part, 1.2 parts, 1.5 parts, 1.8 parts, 2.0 parts, 2.2 parts, 2.5 parts, 2.8 parts, 3.0 parts parts or ranging of any two values.

The encapsulation composition provided in the present invention forms an encapsulation adhesive film by means of a coupled macromolecular light-conversion agent cooperating with a matrix resin and an optional auxiliary agent. The coupled macromolecular light-conversion agent is stably dispersed in the encapsulation adhesive film, and is not easy to migrate. The spectral response range of a solar cell can be effectively expanded, and the photoelectric conversion efficiency of the solar cell is improved.

### [Matrix resin]

In the present invention, the matrix resin is a common resin in the art, including but not limited to any one or more of an EVA resin, a POE resin, a PVB resin, an ethylene-acrylate resin, an ethylene-methacrylate resin, and liquid silicone rubber. The above EVA resin refers to an ethylene-vinyl acetate copolymer; POE resin refers to polyolefin elastomers; PVB resin refers to polyvinyl butyral resin.

### [Hindered amine stabilizer]

In the present invention, hindered amine stabilizers are common stabilizers in the art, and include, but are not limited to, 3,5-di-tert-butyl-4-hydroxy-hexadecyl benzoate, bis-2,2,6,6-tetramethyl-piperidinol tris(1,2,2,6,6-pentamethyl-4-piperidinyl) phosphite sebacic acid, bis-1-decyloxy-2,2,6,6-tetramethyl-piperidin-4-ol sebacic acid, polymers of succinic acid and 4-hydroxy-2,2,6,6-tetramethyl-1-piperidinol, N, any one or more of polymers of N'-bis(2,2,6,6-tetramethyl-4-piperidyl)1,6-hexanediamine, morpholine-2,4,6-trichloro-1,3,5-triazine.

### [Organic peroxide]

In the present invention, organic peroxides are common organic peroxides in the art, including, but not limited to, any one or more of diacyl peroxide, dialkyl peroxide, peroxyester, and peroxyketal.

The peroxyester includes any one or combination of 3,3-bis(t-butylperoxy)butyrate, ethyl 3,3-bis(t-amylperoxy)butyrate, t-butyl peroxybenzoate, t-butyl peroxyacetate, t-butyl peroxyisopropylcarbonate, t-butyl peroxy-3,5,5-trimethylhexanoate, t-butyl peroxy-2-ethylhexylcarbonate, n-butyl 4,4-bis(t-butylperoxy)valerate, t-amyl peroxybenzoate, t-amyl peroxyacetate, t-amyl peroxy-3,5,5-trimethylhexanoate, t-amyl peroxy-2-ethylhexylcarbonate, t-amyl peroxyisobutyrate, 2,5-dimethyl-2,5-bis(benzoyl peroxy)-hexane.

The diacyl peroxide includes any one or combination of benzoyl peroxide, lauric acid peroxide, and capric acid peroxide.

The peroxyketals include any one or combination of 2,2-bis(tert-butylperoxy)butane, 1,1-bis(tert-butylperoxy)cyclohexane, 1,1-bis(tert-butylperoxy)-3,3,5-trimethylcyclohexane, 1,1-bis(tert-amylperoxy)cyclohexane, 1,1-bis(tert-amylperoxy)-3,3,5-trimethylcyclohexane.

Such dialkyl peroxides include dicumyl peroxide and/or 2,5-dimethyl-2,5-bis-(tert-butylperoxy)hexane.

### [Crosslinking coagent]

In the present invention, a crosslinking coagent is a common crosslinking coagent in the art, and comprises but is not limited to any one or more of allyl isocyanurate, triallyl cyanurate, trimethylolpropane triacrylate, trimethylolpropane trimethacrylate and pentaerythritol triacrylate.

### [Tackifier]

In the present invention, a tackifier is a tackifier commonly used in the art, including but not limited to any one or more of γ-aminopropyltriethoxysilane, γ-methacryloxypropyltrimethoxysilane, γ-(2,3-epoxypropoxy)propyltrimethoxysilane, vinyltrimethoxysilane, vinyltriethoxysilane, N-(β-aminoethyl)-γ-aminopropyltrimethoxysilane, γ-glycidoxypropyltrimethoxysilane, 3-aminopropyltrimethoxysilane, vinyltriethoxysilane, vinyltris(β-methoxy.

In a fourth exemplary embodiment of the present disclosure, an encapsulation adhesive film is further provided. The material of the encapsulation adhesive film includes the coupled macromolecular light-conversion agent provided in the first exemplary embodiment or the encapsulation composition provided in the third exemplary embodiment.

The encapsulation adhesive film provided in the present invention comprises a coupled macromolecular light-conversion agent. The coupled macromolecular light-conversion agent is stably dispersed in the encapsulation adhesive film, and is unlikely to migrate. The spectral response range of a photovoltaic module can be effectively expanded, and the photoelectric conversion efficiency of the photovoltaic module can be improved.

In a fifth exemplary embodiment of the present disclosure, a photovoltaic module is further provided. The photovoltaic module includes an encapsulation adhesive film. A material of the encapsulation adhesive film includes the coupled macromolecular light-conversion agent provided in the first exemplary embodiment or the encapsulating composition provided **in** the second exemplary embodiment.

The encapsulation adhesive film used in the photovoltaic module provided in the present invention contains a coupled macromolecular light-conversion agent. The coupled macromolecular light-conversion agent is stably dispersed in the encapsulation adhesive film, and is not easy to migrate. The spectral response range of the photovoltaic module can be effectively broadened, and the photoelectric conversion efficiency of the photovoltaic module can be improved.

Beneficial effects of the present disclosure will be further described below with reference to the embodiments and comparative examples.

### Example 1

The present embodiment provides a coupled macromolecular light-conversion agent, the structural formula thereof being as shown in formula (1):

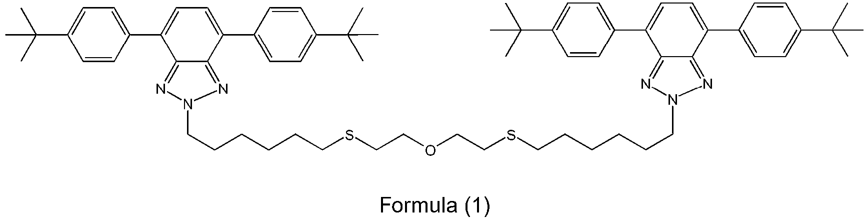

The preparation method thereof comprises the following steps:
4,7-Bis(4-tert-butylphenyl)-(6-hexenyl)-2H-benzotriazole (9.31 g, 20 mmol) was dissolved completely in 50 mL of cyclohexane, bis(2-mercaptoethyl) ether (1.24 g, 9 mmol) was added, the initiator lauroyl LP peroxide (0.30 g) was added and the mixture was heated and stirred at 75°C for 12 hours. After the reaction is completed, the reaction solution is cooled to room temperature, the solvent is removed by rotary evaporation, column chromatography is separated, and the product is dried overnight under vacuum at 30°C to obtain 8.4 g of the compound 1 with a calculated yield of 87%.

### Example 2

The present embodiment provides a coupled macromolecular light-conversion agent, the structural formula thereof being as shown in formula (2) below:

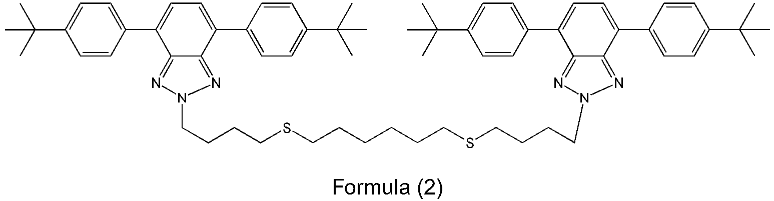

The preparation method thereof comprises the following steps:
4,7-Bis(4-tert-butylphenyl)-(4-butanethiol)-2H-benzotriazole (9.43 g, 20 mmol) was dissolved completely in 50 mL of cyclohexane, 1,6-hexadiene (0.74 g, 9 mmol) was added, and the initiator lauroyl LP peroxide (0.30 g) was added and stirred at 75°C for 12 hours. After the reaction is completed, the reaction solution is cooled to room temperature, the solvent is removed by rotary evaporation, column chromatography is separated, and the product is dried overnight under vacuum at 30°C to obtain 7.8 g of a compound at a calculation yield of 85%.

### Example 3

The present embodiment provides a coupled macromolecular light-conversion agent, the structural formula thereof being as shown in formula (3) below:

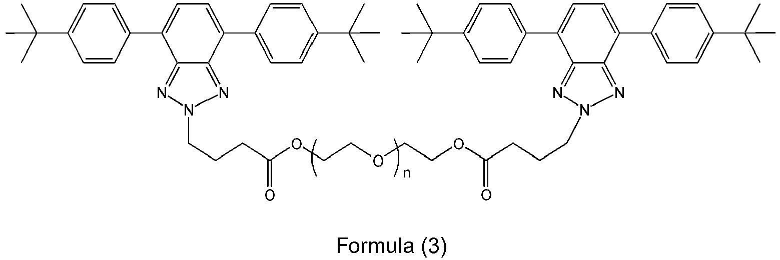

The preparation method thereof comprises the following steps:
4,7-Bis(4-tert-butylphenyl)-(butyrylcarboxylic acid group)-2H-benzotriazole (9.39 g, 20 mmol) was completely dissolved in 50 mL of dichloromethane, polyethylene glycol (18 g, Mn = 2000 g/mol) was added, catalyst 4-dimethylaminopyridine (0.9 g) and dehydrated agent dicyclohexylcarbodiimide (3.8 g) were further added, and the mixture was heated and stirred at 25°C for 24 hours. After the reaction is completed, filtering is performed, the filtrate is washed with water multiple times, anhydrous sodium sulfate is added for water absorption, filtering is performed, the filtrate is distilled by rotary evaporation to remove the solvent, the product is dried overnight under vacuum at 30°C, 25 g of a compound is obtained, and the calculation yield is 85%.

### Example 4

The present embodiment provides a coupled macromolecular light-conversion agent, the structural formula thereof being as shown in formula (4):

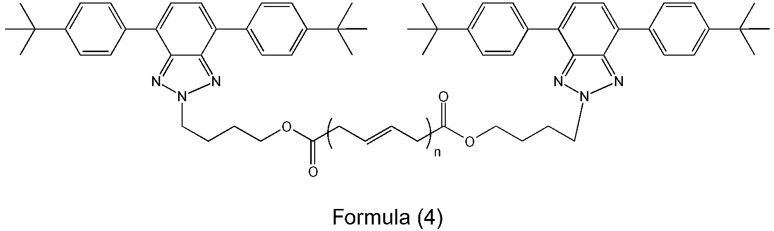

The preparation method thereof comprises the following steps:
4,7-bis(4-tert-butylphenyl)-(4-butanol)-2H-benzotriazole (9.11 g, 20 mmol) is completely dissolved in 50 mL of dichloromethane, terminal carboxyl polybutadiene (27 g, Mn = 3000 g/mol) is added, catalyst 4-dimethylaminopyridine (0.90 g) and dehydrated agent dicyclohexylcarbodiimide (3.6 g) are added, and the mixture is heated and stirred at 25°C for 24 hours. After the reaction is completed, filtration is performed, the filtrate is washed with water multiple times, 500 mL of ethyl acetate is added thereto to precipitate a macromolecular light-conversion agent, then filtration is performed, the filtrate is discarded, the residue is washed with 100 mL of ethyl acetate three times, dried overnight under vacuum at 30°C to obtain 31 g of a compound, and the calculated yield is 80%.

### Example 5

The present embodiment provides a coupled macromolecular light-conversion agent, the structural formula thereof being as shown in formula (5):

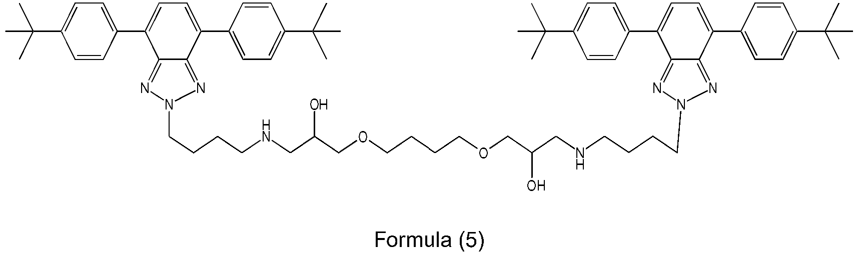

The preparation method thereof comprises the following steps:
4,7-Bis(4-tert-butylphenyl)-(4-butylamino)-2H-benzotriazole (9.09 g, 20 mmol) was completely dissolved in 50 mL of cyclohexane, 1,4-butanediol diglycidyl ether (0.74 g, 9 mmol) was added, and triethylamine (0.09 g) as a catalyst was further added to the mixture, which was then heated and stirred at 75°C for 24 hours. After the reaction was completed, the reaction solution was cooled to room temperature, the solvent was removed by rotary evaporation, column chromatography was separated, and the product was dried overnight under vacuum at 30°C to obtain 7.2 g of a compound at a calculation yield of 81%.

### Example 6

The present embodiment provides a coupled macromolecular light-conversion agent, the structural formula thereof being as shown in formula (6) below:

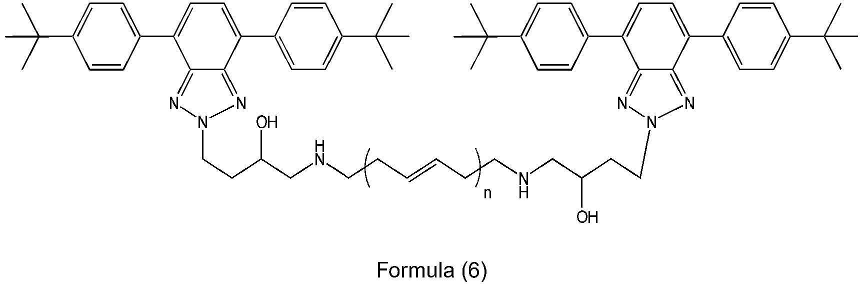

The preparation method thereof comprises the following steps:
4,7-Bis(4-tert-butylphenyl)-(3,4-butylepoxy)-2H-benzotriazole (9.07 g, 20 mmol) was completely dissolved in 50 mL of cyclohexane, terminal-amine polybutadiene (36 g, Mn = 4000 g/mol) was added, catalyst triethylamine (0.09 g) was added, and then heated and stirred at 75°C for 24 hours. After the reaction is completed, filtration is performed, the filtrate is washed with water multiple times, 500 mL of ethyl acetate is added thereto to precipitate a macromolecular light-conversion agent, then filtration is performed, the filtrate is discarded, the residue is washed with 100 mL of ethyl acetate three times, dried overnight under vacuum at 30°C to obtain 34 g of a compound, and the calculated yield is 78%.

### Example 7

The present embodiment provides a coupled macromolecular light-conversion agent, the structural formula thereof being as shown in formula (7):

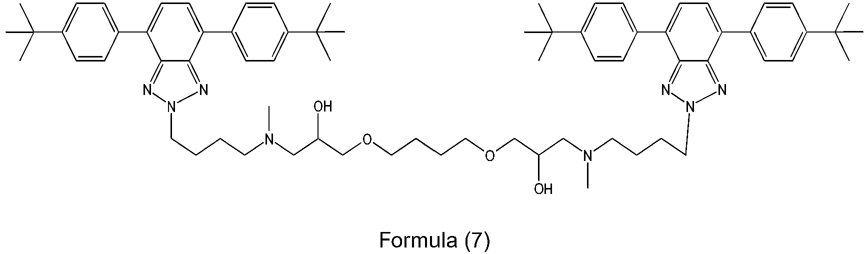

The preparation method thereof comprises the following steps:
4,7-bis(4-tert-butylphenyl)-(4-butylmethylamino)-2H-benzotriazole (9.37 g, 20 mmol) is completely dissolved in 50 mL of cyclohexane, 1,4-butanediol diglycidyl ether (0.74 g, 9 mmol) is added, and triethylamine (0.09 g) as a catalyst is added and stirred at 75°C for 24 hours. After the reaction is completed, the reaction solution is cooled to room temperature, the solvent is removed by rotary evaporation, column chromatography is separated, and the product is dried overnight under vacuum at 30°C to obtain 6.9 g of a compound at a calculated yield of 75%.

### Example 8

The present embodiment provides a coupled macromolecular light-conversion agent, the structural formula thereof being as shown in formula (8):

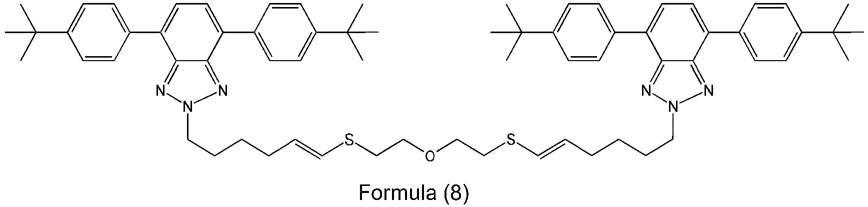

The preparation method thereof comprises the following steps:
4,7-bis(4-tert-butylphenyl)-(6-hexynyl)-2H-benzotriazole (9.27 g, 20 mmol) was completely dissolved in 50 mL of cyclohexane, bis(2-mercaptoethyl) ether (1.24 g, 9 mmol) was added, and initiator azodiisobutyronitrile (0.30 g) was added and then heated and stirred at 75°C for 12 hours. After the reaction is completed, the reaction solution is cooled to room temperature, the solvent is removed by rotary evaporation, column chromatography is separated, and the product is dried overnight under vacuum at 30°C to obtain 8.0 g of a compound at a calculation yield of 83%.

### Example 9

The present embodiment provides a coupled macromolecular light-conversion agent, the structural formula thereof being as shown in formula (9) below:

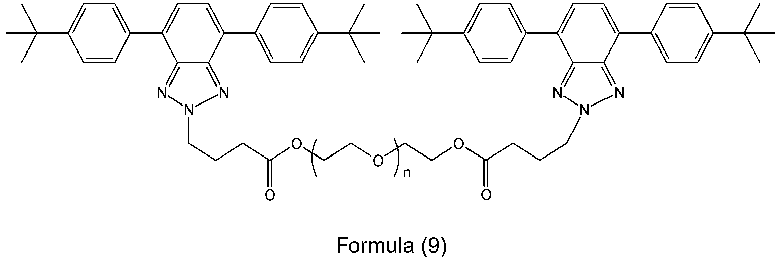

The preparation method thereof comprises the following steps:
4,7-Bis(4-tert-butylphenyl)-(butyrylcarboxylic acid group)-2H-benzotriazole (9.39 g, 20 mmol) was completely dissolved in 5000 mL of dichloromethane, polyethylene glycol (810 g, Mn = 90000 g/mol) was added, catalyst 4-dimethylaminopyridine (0.9 g) and dehydrated agent dicyclohexylcarbodiimide (10.0 g) were added, and then heated and stirred at 25°C for 24 hours. After the reaction is completed, filtering is performed, the filtrate is washed with water multiple times, anhydrous sodium sulfate is added for water absorption, filtering is performed, the filtrate is distilled by rotary evaporation to remove the solvent, the product is dried overnight under vacuum at 30°C, and 795 g of a compound is obtained with a calculated yield of 97%.

It should be noted that, the difference between the compound of formula (9) and the compound of formula (3) lies in that the value of n is different, and the value of n represents the polymerization degree of polyethylene glycol. Since the molar masss of the polyethylene glycol used in embodiment 3 and the present embodiment are completely different, the compound of formula (9) and the compound of formula (3) are also different.

### Comparative Example 1

The present comparative example provides an organic small molecule light conversion agent, which is 4,7-bis(4-tert-butylphenyl)-(6-hexenyl)-2H-benzotriazole (TAH).

### Example 10

This example provides a packaging composition. The packaging composition comprises, in parts by mass, 100 parts of an EVA resin (manufactured by Honghua Dahl Co., Ltd., Model: E280PV), 0.5 parts of an organic peroxide tert-amyl peroxy 2-vinylhexyl carbonate (manufactured by Arkema Co., Ltd., Luperox TAEC), 0.6 parts of a crosslinking coagent triallyl isocyanurate TAIC, and 0.1 part of a macromolecular light conversion agent. The macromolecular light-conversion agent is the coupled macromolecular light-conversion agent provided in embodiment 1.

### Example 11

This example differs from Example 10 in that the amount of the macromolecular light-conversion agent is 1 part.

### Example 12

This example differs from Example 10 in that the amount of the macromolecular light-conversion agent is 0.01 part by weight.

### Examples 13 to 19

Examples 13-19 differ from Example 10 in that the coupled macromolecular light-conversion agent provided in Examples 2-8 is used as the macromolecular light-conversion instead of the coupled macromolecular light-conversion agent provided in Example 1.

### Example 20

This example differs from Example 10 in that the macromolecular light-conversion agent provided in Example 9 is used to replace the coupled macromolecular light-conversion agent provided in Example 1 as a macromolecular, and the amount of macromolecular light-conversion agent provided in Example 9 is 1 part.

### Comparative example 2

The difference between the present comparative example and Example 10 lies in that the coupled macromolecular light-conversion agent provided in Example 1 is replaced with the small molecule light-conversion agent provided in Comparative Example 1 as the light-conversion agent.

### Test Example

The encapsulating compositions provided in the embodiments and comparative examples are respectively prepared into an encapsulation adhesive film, and the specific steps comprise: fully mixing components of the encapsulating composition via a mixer, and melt-extruding same via a screw extruder, so as to obtain an encapsulating film of 450 g/m².

The above-mentioned packaging films are respectively assembled into a solar module, specifically comprising the following steps: successively arranging a front plate glass, the described packaging films, a silicon heterojunction cell (a sublimation cell sheet), an ultraviolet transmission packaging adhesive film (Foster F406P) and a rear plate glass in a bottom-up order, obtaining a laminate, placing the laminate in a vacuum laminator, and pressing same at 145°C for 15 min to obtain a solar module, except for different upper layer packaging films, all the components prepared each time are controlled consistently.

Power retention test method: The above-mentioned solar assembly was left at 25°C for 6 h, and then the power was measured with a solar power simulator (Gsola GIV-20A), the initial power meter being P1. Afterwards, the solar assembly is placed in a UV ageing box for UV irradiation, the total irradiation dose being 300 kWh, the power after the irradiation is measured and recorded as P2, and the power retention rate = P2/P1 * 100% is calculated.

The method for testing the migration distance of a light-conversion agent: the newly prepared solar assemblies above were respectively allowed to stand at 25°C for 6 h, and then were baked in an oven at 120°C for 48 h, 144 h and 288 h. After that, the migration distance (unit/cm) of the light-conversion agent from the edge of the cell sheet to the back surface of the cell sheet was evaluated. The larger the migration distance, the severer the migration of the light-conversion agent.

The results are shown in Table 1 below.

**Table 1**

| | Initial power P1(W) | Post-irradiation power P2 (W) | Power retention rate (%) | Migration distance (48h) | Migration distance (144h) | migration distance (288 h) |
|---|---|---|---|---|---|---|
| Example 10 | 35.1 | 33.9 | 96.6 | 0.2 | 0.8 | 1.3 |
| Example 11 | 34.2 | 31.6 | 92.4 | 0.9 | 1.4 | 1.9 |
| Example 12 | 32.1 | 29.1 | 90.7 | 0.2 | 0.8 | 1.2 |
| Example 13 | 35.1 | 33.1 | 94.3 | 0.4 | 0.8 | 1.6 |
| Example 14 | 34.7 | 33.5 | 96.5 | 0.3 | 0.8 | 1.5 |
| Example 15 | 34.9 | 33.5 | 96.0 | 0.3 | 0.9 | 1.4 |
| Example 16 | 34.6 | 33.2 | 96.0 | 0.5 | 1.0 | 1.5 |
| Example 17 | 35.0 | 33.7 | 96.3 | 0.2 | 0.7 | 1.1 |
| Example 18 | 34.7 | 33.2 | 95.7 | 0.3 | 0.9 | 1.3 |
| Example 19 | 34.8 | 33.3 | 95.7 | 0.4 | 0.9 | 1.5 |
| Example 20 | 33.2 | 30.3 | 91.3 | 0.2 | 0.7 | 1.1 |
| Comparative example 2 | 31.9 | 26.2 | 82.1 | 2.3 | 3.9 | 4.8 |

From the above description, it can be seen that the above embodiments of the present invention achieve the following technical effects: the encapsulation composition provided by the present invention forms an encapsulation adhesive film by compounding a coupled macromolecular light-conversion agent with a matrix resin and an optional auxiliary agent, the coupled macromolecular dispersive agent is stably dispersed in the encapsulation adhesive film, and is unlikely to migrate, which can effectively broaden the spectral response range of the solar cell, to improve the photoelectric conversion efficiency of a solar cell.

The foregoing descriptions are merely exemplary embodiments of the present invention, but are not intended to limit the present invention. For those skilled in the art, the present invention may have various modifications and variations. Any modifications, equivalent replacements, improvements and the like made within the spirit and principle of the present invention shall fall within the scope of protection of the present invention.

## Claims

1. A coupled macromolecular light-conversion agent, wherein the coupled macromolecular light-conversion agent comprises a first light-conversion agent and a second light-conversion agent, and the first light-conversion agent and the second light-conversion agent are coupled; the first light-conversion agent and the second light-conversion agent are each independently a benzotriazole compound, and the benzotriazole compound has a structure as shown in formula (I): 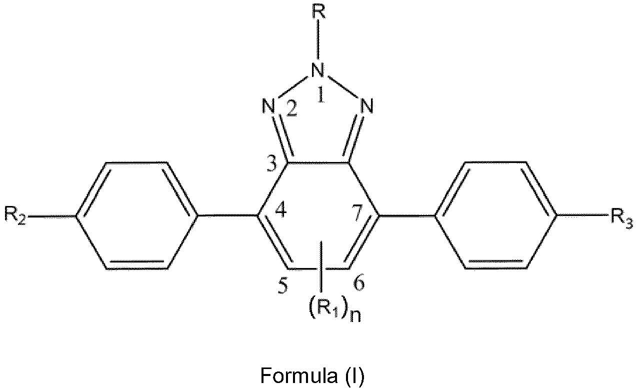
wherein n is 1 or 2, R₁ is selected from H, a substituted or unsubstituted C1 to C20 alkyl group, or a first group;
R₂, R₃ are each independently selected from H, a substituted or unsubstituted C1 to C20 alkyl group;
R and the first group are each independently R₅-R₄*, wherein R₅ represents a terminal group, and R₅ is at least one selected from the group consisting of mercapto group, carboxyl group, hydroxyl group, primary amino group, secondary amino group, epoxy group, vinyl group and ethynyl group; R₄ is selected from a direct bond, a substituted or unsubstituted C1 to C20 alkyl group, and C and H in the alkyl group may be optionally substituted with O.

2. The coupled macromolecular light-conversion agent according to claim 1, wherein the R is selected from *CH₂-(CH₂)₅-CH=CH₂, *CH₂-CH₂COO-CH=CH₂, *CH₂-(CH₂)₂-COOH, *CH₂-(CH₂)₂-CH₂OH, *CH₂-(CH₂)₃-SH, *CH₂-(CH₂)₃-NH₂;
and/or R₁ is selected from H, a C1-C6 alkyl group, *CH₂-(CH₂)₅-CH=CH, CH₂-(CH₂)₂-COOH, *CH₂-(CH₂)₂-CH₂OH, *CH₂-(CH₂)₃-NH₂, *CH₂-(CH₂)₃-SH;
and/or, each of R₂ and R₃ is independently selected from H or C1-C6 alkyl.

3. The coupled macromolecular light-conversion agent according to claim 1, wherein the number-average molecular weight of the coupled macromolecular light-conversion agent is 1000-100000 g/mol;
and/or, the maximum absorption wavelength of the coupled macromolecular light-conversion agent is between 300-400 nm, and the maximum emission wavelength is greater than 400 nm.

4. The coupled macromolecular light-conversion agent according to any one of claims 1 to 3, wherein the first light-conversion agent and the second light-conversion agent are each independently selected from at least one of the following compounds:

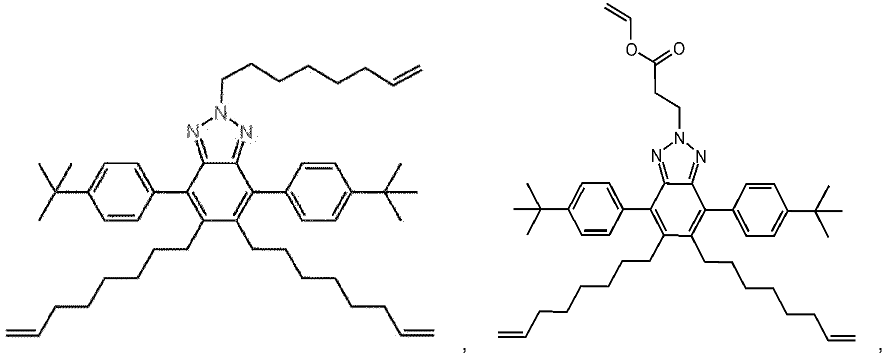

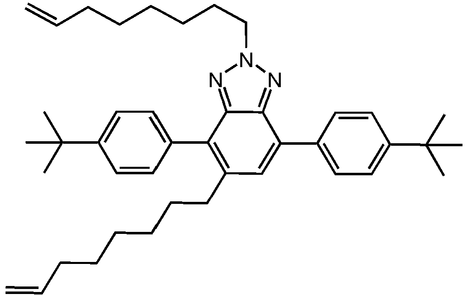

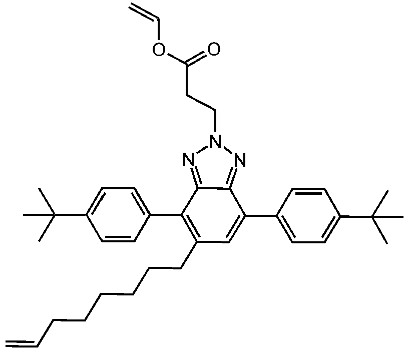

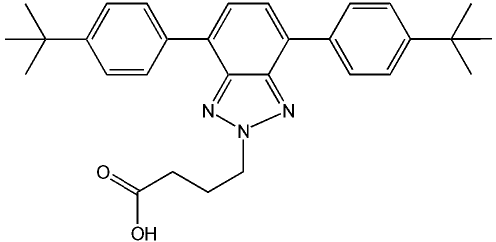

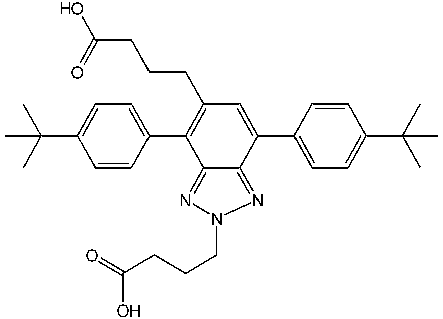

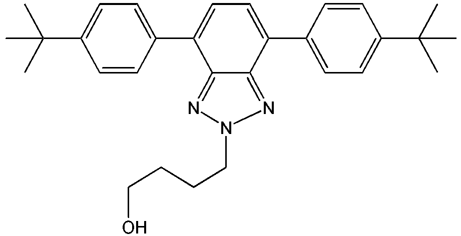

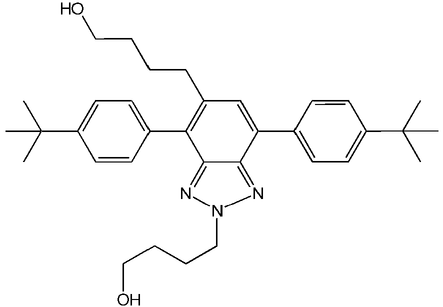

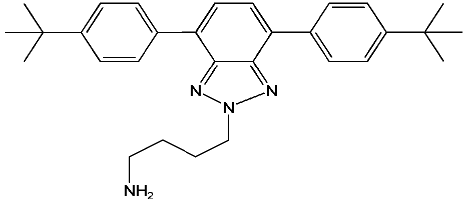

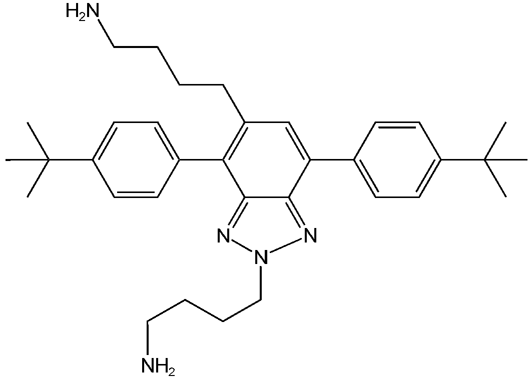

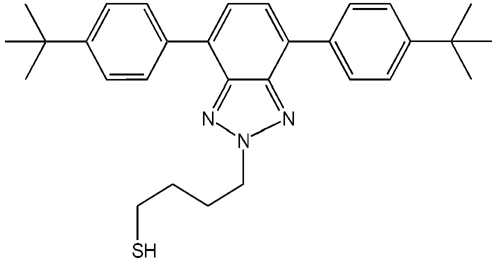

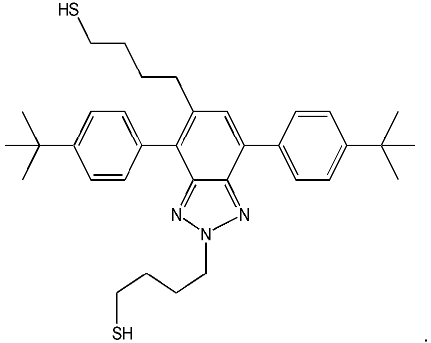

5. A preparation method of the coupled macromolecular light-conversion agent according to any one of claims 1 to 4, wherein the preparation method comprises:
step S1, respectively dissolving the first light-conversion agent and the second light-conversion agent in a solvent, so as to obtain a first light-conversion agent solution and a second light-conversion agent solution;
step S2, mixing the first light-conversion agent solution, the second light-conversion agent solution, a coupling agent and an optional initiator for a coupling reaction to obtain the coupled macromolecular light-conversion agent.

6. The preparation method according to claim 5, wherein in the step S2, the coupling agent is selected from at least one of bis(2-mercaptoethyl)ether, 4,4'-bis(mercaptomethyl)biphenyl, C2-C10 glycol, polyethylene glycol, polytetrahydrofuran, poloxamer, polyvinyl alcohol, terminal(poly)hydroxypolybutadiene, terminal(poly)hydroxypolyethylene, terminal hydroxyl liquid nitrile rubber, C2-C10 dicarboxylic acid, terminal carboxyl polybutadiene, terminal carboxyl polyethylene, terminal carboxyl nitrile rubber, polyacrylic acid, polymethacrylic acid, C2-C10 diamine, polyether amine, terminal amino liquid nitrile rubber, terminal amino polybutadiene, terminal amino polyethylene, 1,4-butanediol glycidyl ether, glycidyl ether, epoxy resin, C4-C18 diene, polybutadiene, ethylene-propylene-diene ternary rubber, and styrene-butadiene rubber.

7. An encapsulation composition, wherein the encapsulation composition comprises the following components in parts by mass: 100 parts of a matrix resin, 0.01-1 part of a macromolecular light-conversion agent, 0-1 part of a hindered amine light stabilizer, 0-2 parts of an organic peroxide, 0-3 parts of a crosslinking coagent, and 0-3 parts of a tackifier; the macromolecular light-conversion agent is the coupled macromolecular light-conversion agent of any one of claims 1 to 4.

8. The encapsulation composition according to claim 7, wherein the matrix resin comprises at least one of an EVA resin, a POE resin, a PVB resin, an ethylene-acrylate resin, an ethylene-methacrylate resin, and a liquid silicone rubber.

9. The encapsulation composition according to claim 7, wherein the hindered amine light stabilizer comprises 3,5-di-tert-butyl-4-hydroxy-hexadecyl benzoate, tris(1,2,2,6,6-pentamethyl-4-piperidinyl) phosphite, bis(2,2,6,6-tetramethylpiperidinol) sebacate, bis-1-decyloxy-2,2,6,6-tetramethylpiperidin-4-ol sebacic acid, a polymer of succinic acid and 4-hydroxy-2,2,6,6-tetramethyl-1-piperidinol, at least one of polymers of N'-bis(2,2,6,6-tetramethyl-4-piperidyl)1,6-hexanediamine, morpholine-2,4,6-trichloro-1,3,5-triazine;
and/or the organic peroxide comprises at least one of diacyl peroxide, dialkyl peroxide, peroxyester, and peroxyketal;
and/or, the crosslinking coagent comprises at least one of triallyl isocyanurate, triallyl cyanurate, trimethylolpropane triacrylate, trimethylolpropane trimethacrylate, pentaerythritol triacrylate;
and/or the tackifier comprises at least one of γ-aminopropyltriethoxysilane, γ-methacryloxypropyltrimethoxysilane, γ-(2,3-epoxypropoxy)propyltrimethoxysilane, vinyltrimethoxysilane, vinyltriethoxysilane, N-(β-aminoethyl)-γ-aminopropyltrimethoxysilane, γ-glycidoxypropyltrimethoxysilane, 3-aminopropyltrimethoxysilane, vinyltriethoxysilane, vinyltris(β-methoxyethoxy silane), and allyltrimethoxysilane.

10. An encapsulation adhesive film, wherein the material of the encapsulation adhesive film comprises the coupled macromolecular light-conversion agent as claimed in any one of claims 1 to 4 or the encapsulation composition as claimed in any one of claims 7 to 9.

11. A photovoltaic module, wherein the photovoltaic module comprises an encapsulation adhesive film, and the material of the encapsulation adhesive film comprises the coupled macromolecular light-conversion agent of any one of claims 1 to 4 or the encapsulation composition of any one of claims 7 to 9.
